# EUROPEAN PATENT SPECIFICATION

(11) **EP 0 755 386 B1**
(45) Date of publication and mention of the grant of the patent: **05.06.2002**
(21) Application number: 95917310.5
(22) Date of filing: 11.04.1995
(51) Int. Cl.: C07D 277/58, A61K 31/425, A61K 9/06, A61K 9/20

(54) **BENZAMIDE DERIVATIVE, COMPOSITIONS CONTAINING SAID DERIVATIVE AND USE THEREOF**
BENZAMIDE-DERIVATE, ZUBEREITUNGEN DIE SIE ERHALTEN UND IHRE ANWENDUNG
DERIVE DE BENZAMIDE, COMPOSITIONS LE CONTENANT ET SON UTILISATION

(30) Priority: 13.04.1994 US 227033; 08.09.1994 US 301407; 06.02.1995 US 383855
(43) Date of publication of application: 29.01.1997
(73) Proprietor: Romark Laboratories, L.C., Tampa, FL 33607 (US)
(72) Inventor: Rossignol, Jean François, Clearwater, FL 34622 (US)
(74) Representative: Callewaert, Jean
(86) International application number: EP9501334
(87) International publication number: WO9528393

(56) References cited:
- FR-A- 1 306 603
- FR-A- 2 240 010
- FR-A- 2 435 905
- FR-M- 716
- US-A- 4 315 018
- ANTIMICROBIAL AGENTS AND CHEMOTHERAPY, vol. 12, no. 3, September 1977 WASHINGTON DC (USA), pages 353-356, M. DYMICKY ET AL 'Inhibition of Clostridium botulinum by 5-nitrothiazoles'
- EUROPEAN JOURNAL OF MEDICINAL CHEMISTRY,CHIMICA THERAPEUTICA., vol. 13, no. 6, 1978 PARIS FR, pages 539-543, RAYMOND CAVIER ET AL 'Recherches sur les dérivés nitrés d'intérêt biologique'

## Description

The present invention relates to a new compound of formula I 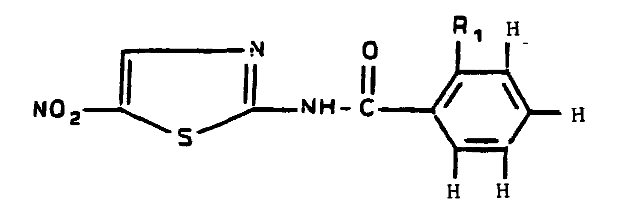 with the symbol R₁ representing OH,
to a pharmaceutical composition containing the said compound, and to the use of said compound as anti-parasital, anti-bacterial, anti-fungal agent, anti viral agent.

### The Prior Art

Nitrothiazole compound PH 5776 (2-(acetolyloxy)-N-(5-nitro 2-thiazolyl) benzamide) is a compound of formula I 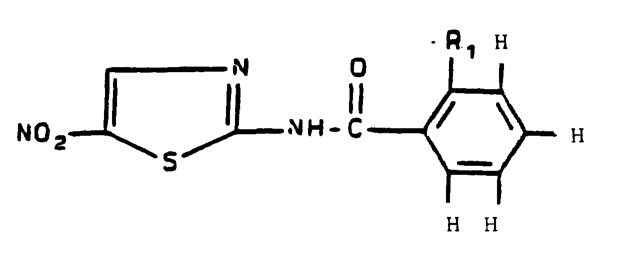 in which R₁ = O-COCH₃.

The preparation and uses of this compound (called hereafter Compound B - formula II) are disclosed in US 3,950,351, as well as in publication made by Applicant.

In US 3,950,351, the compound B of formula II is prepared by reacting 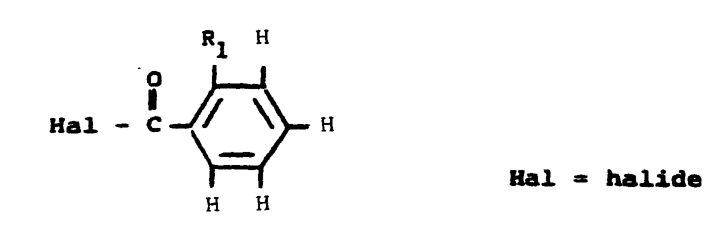 with

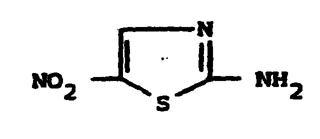

This reaction is not suitable for the preparation of pure compound of formula I 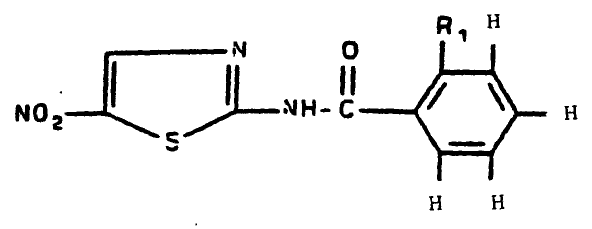 in which the symbol R₁ represents OH.

Moreover, contrary to what could be expected from the prior art, i.e. that the presence of an acyloxy group was necessary for rendering the compound active and efficient against bacteria, parasites, ..., it has now been found that the compound of formula I 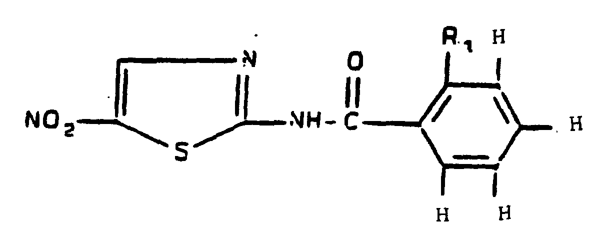 with the symbol R₁ representing OH,
had an excellent efficiency against parasites, bacteria, fungus although it does not contain an acyloxy group. The said compound I had a substantially immediate action against parasite, fungus, bacteria.
It has also been found that said compound was active against viruses.

It has also been found that composition containing the said compound I also advantageously contains a wetting agent. Most preferred compositions are those containing a wetting agent and a starch derivative.

Tests made by applicant have also shown that by using simultaneously the compound of the invention and a wetting agent, the efficiency of the compound is drastically increased and that by using such a mixture, it is possible to treat affections of the lower abdomen, such as intestinal affections (diarrhea), gastrointestinal infections, enteric infections, sexually transmitted infections, vaginal infections and urinogenital infections.

The invention relates therefore also to a composition for combatting affections of the lower abdomen, said composition containing :
* an effective amount of an active agent of formula I 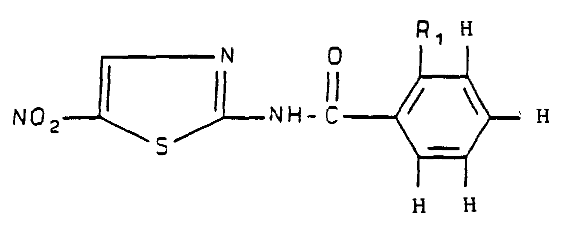
* and a wetting agent,
said composition comprising preferably also a starch derivative.

### Brief Description of the Invention

The present invention relates to a new compound A of formula I 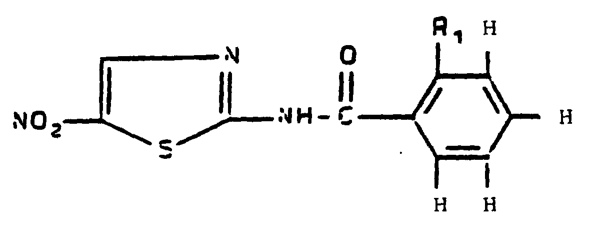 in which the symbol R₁ represents OH.

The invention relates also to a pharmaceutical composition comprising as active agent a compound of formula I in which R₁ = OH (formula III).

According to an embodiment, the composition comprises, as active agent, a mixture of Compound A of formula 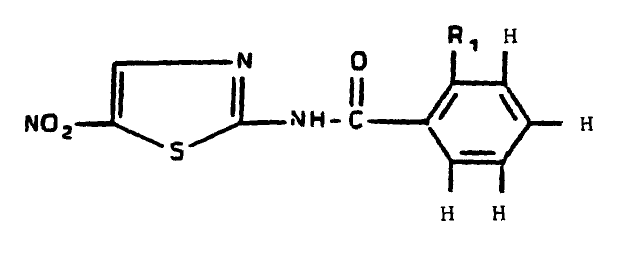 with R₁ = OH and R₂ = R₃ = R₄ = R₅ = H
and

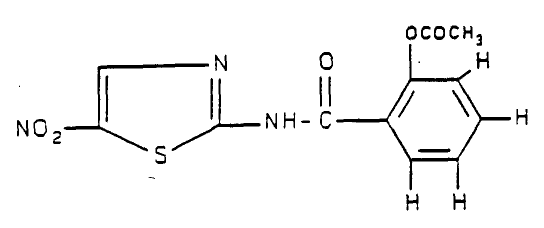

Such a composition combines a substantially immediate action against parasite, fungus, bacteria, virus or a substiantially immediate treatment of intestinal affection and a some what retarded action or treatment.

Such a composition is thus suitable for treating human affections or for preventing human affections, such as parasitic infections, bacterial infections, fungal infections, diarrhea and other intestinal affections, such as affections due to viruses.

In said composition, the weight content of Compound A of formula III 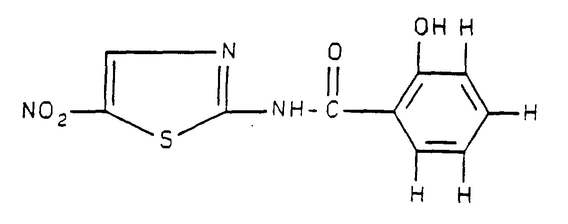 with respect to the weight of the mixture of compound A of formula III 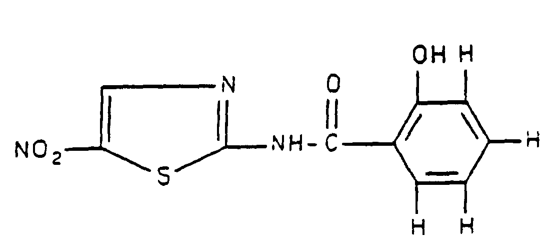 and compound B of formula 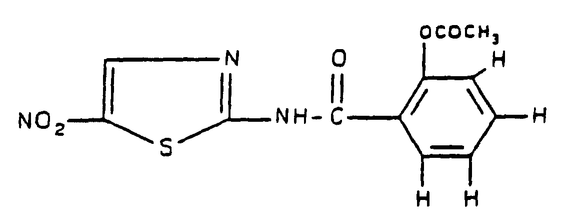 is comprised between 0.5 and 20 %, preferably between 0.5 and 10 %, more preferably between 0.5 and 5 %.

The invention relates also to the use of a compound according to the invention, especially a composition according to the invention as anti-parasital agent, anti-bacterial agent, anti-fungal agent, antiviral agent.

The composition may contain further active agents, such as anthelmintic agent, and anti-viral agent, ...

The composition may also contain a wetting agent and/or a starch derivative and/or an excipient.

A composition, preferably a solid composition, for oral administration for combatting affections or infections of the lower abdomen, preferably intestinal and vaginal conditions, contains :
* an effective amount of Compound B of formula 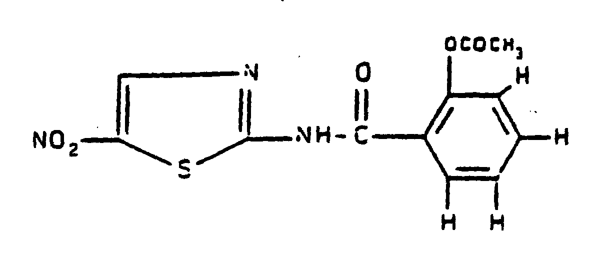
* a wetting agent, and possibly, but preferably
* at least one starch derivative.

The latter composition may also contain other active agents, for example an anthelmintic agent such as febantel, praziquantel, levamisole, albendazole, oxfendazole, moxidectin, ivermectin, milbemycins, etc.

The preparation and use of compound B is disclosed in US 3,950,351 and US 4,315,018.

The wetting agent present in the composition containing compound A or a mixture of compound A and compound B is advantageously an anionic surfactant, and is preferably selected among the group consisting of sugar esters, polyoxyethylene, polyoxypropylene, anhydrohexitol derivatives, fatty alkanol amides, fatty amine oxides, sucrose, mannitol, sorbitol, lecithins, polyvinylpyrrolidones, fatty acid esters, glycerides of sucrose, esters of xylose, polyoxyethylene glycerides, esters of fatty acids and polyoxyethylene ether of fatty alcohols and polyoxyethylene fatty acid esters of sorbitan, polyoxyethyelene esters of fatty acids of sorbitan, glyceridepolyglycides, esters of alcohol polyglycides and mixture thereof.

Particularly suitable wetting agents are saccharose distearate, PVP (polyvinylpyrrolidone), etc. The composition according to the invention contains preferably a starch derivative, especially a carboxy derivative of starch, such as carboxymethyl starch, natrium derivative thereof or a salt thereof.

The composition contains, for example, up to 20 % by weight, advantageously 1 to 10 % by weight of surfactant with respect to the weight of active agent(s), and up to 20 % by weight, advantageously 1 to 10 % by weight of starch derivative with respect to the weight of active agent(s).

The invention relates also to a galenic formulation for oral administration against viruses and/or for combatting affections of the lower abdomen, such as intestinal, vaginal or urogenital conditions or disorders, said galenic formulation comprising a core containing a composition containing :
* an effective amount of an active agent of compound A and/or compound B,
* a wetting agent, and possibly, but preferably
* a starch derivative or a salt thereof,
the water content of said composition being less than 25 % by weight, the said core being preferably coated with a membrane. Such a membrane can be a membrane insoluble in the acid gastric medium, but soluble in the intestine.

The preferred compositions, wetting agents, etc of the galenic formulation are those disclosed hereabove for the composition according to the invention.

The invention relates yet to the use of the composition or the galenic formulation for the treatment of diarrhea, for the treatment of liver troubles,...

The invention relates also to an ointment or gel, cream or suppositories for the treatment of organs of the lower abdomen, such as vaginal or urogenital conditions or disorders, disorders of the rectum. The ointment, preferably in the gel form, comprises a compound A of formula III and compound B, a wetting agent, as well as excipient.

The wetting agent is preferably one of those listed above for the composition and/or galenic formulation.

Furthermore, the invention relates to an anti-bacterial composition containing a compound A of formula III and/or compound B, and a wetting agent. Such a composition is active against aerobic, as well anaerobic bacteria, the composition having thus a very large spectra of activity.

The invention relates thus also to a process for killing bacteria or for preventing the presence or growth of bacteria in a medium. In said process, the bacteria or medium is treated with a bactericidal composition according to the invention, for example by spraying the composition on the bacteria, by immersing a support into a medium, etc.

The invention relates also to a process for treating diarrhea affections of animals, preferably humans, in which a composition containing :
* an effective amount of an active agent of compound A and/or of compound B
* a wetting agent
* a starch derivative
is orally administered.

The invention relates furthermore to a food composition such as a food pasta or a yaourt comprising, as preserving agent,
* an effective amount of a mixture of compound A and compound B
* possibly, but advantageously a wetting agent, and possibly, but preferably together with a wetting agent ;
* a starch derivative

### Description of the invention

The preparation of pure compound of formula I 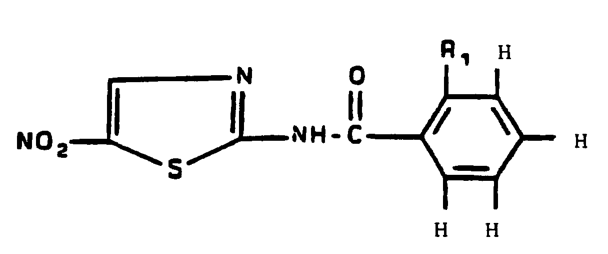 in which the symbol R₁ represents OH.
can be made from compound of formula I 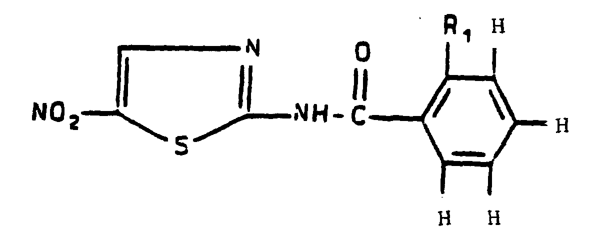 in which the symbol R₁ is an acyloxy group.

The said compound is put in suspension in a weak mixture of hydrochloridric acid and water. The so treated compound is then filtered and washed with water. The washed compound is then possibly dried.

### EXAMPLE OF PREPARATION

### Preparation of compound A

A specific example of preparation is given hereafter :
2 g of 2- (acetolyloxy)-N- (5-nitro 2-thiazolyl) benzamide (i.e. PH 5776) - compound B prepared according to the method disclosed in US 3,950,351 is put in suspension in 20 ml of a 37 % HCl solution. The medium was kept at 50°C during 24 hours and was slowly stirred.

After said treatment, the medium was filtered so as to obtain solid particles. Said particles have then been washed with water until pH 7 and dried in an oven at 50°C.

The resulting product appears as yellow microcristalline needles, the melting point of which was 254°C (melting point measured according to the capillary determination on a Mettler FP apparatus).

The structure identification was carried out by centesimal analysis, UV spectrum , IR spectrum and gas-chromatography-mass spectrum. The results of this identification are :
C10, H7, N3, O4, S1 ; 258

| | | | | |
|---|---|---|---|---|
| Calculated | C 46.51 % | H 2.71 % | N 16.40 % | S 12.40 % |
| found | 45.98 % | 2.63 % | 16.71 % | 12.67 % |

λₘₐₓ = 350 nm (OD = 0.605).

### TEST 1

### Preparation of Composition O

A composition according to the invention has been prepared by mixing PH 5776 and the compound prepared hereabove, the weight content of said compound with respect to the weight of the said compound and PH 5776 being 4 %.

### Composition A1

100 g nitazoxanide (PH 5776) was mixed in 100 ml water with 10 g of polyvinylpyrrolidone (wetting agent) and 5 g carboxymethyl starch. The mixture has been dried under vaccum.

Said composition can then be used for making granules, sachets, tablets or capsule for buccal or oral administration.

### Compositions B1,C1,D1,E1,F1,G1

Compositions similar to composition A1 were prepared, except that only 5 g and 2 g of polyvinyl-pyrrolydone, and 2 g and 1 g carboxymethyl starch were used.

The following table gives the content (g) of nitazoxanide "N", polyvinylpyrrolidone "PVF" and carboxymethyl starch "CS" of the compositions.

| Composition | N (g) | PVP (g) | CS (g) |
|---|---|---|---|
| B1 | 100 | 5 | 5 |
| C1 | 100 | 2 | 2 |
| D1 | 100 | 5 | 2 |
| E1 | 100 | 5 | 1 |
| F1 | 100 | 2 | 1 |
| G1 | 100 | 5 | 0 |

### Compositions H1, I1, J1

Compositions containing nitazoxanide, as well as another active agent have been prepared by preparing an aqueous medium containing PVP and carboxymethyl starch and by adding to said medium nitazoxanide and another active agent. The medium was then dried to obtain a water content lower to 5 %.

| Composition | N g | PVP g | CS g | Other active agent g | Water content % |
|---|---|---|---|---|---|
| H1 | 50 | 5 | 2 | Praziquantel 50 | 2 |
| I1 | 75 | 5 | 2 | Praziquantel 50 | 1 |
| J1 | 75 | 5 | 2 | Praziquantel 50 | 2 |

### Compositions A2 to J2

Compositions similar to compositions A1 to J1 have been prepared.

The compositions A2 to J2 differ from compositions A1 - J1 only by the fact that they contain a mixture of nitazoxanide (PH 5776 - compound B) and compound A of formula : 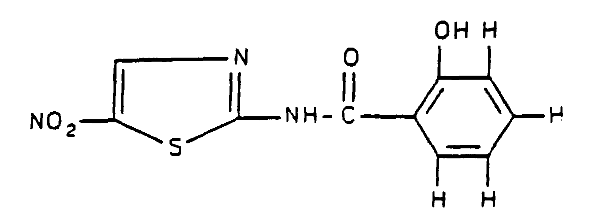 (instead of nitazoxanide only in compositions A1 - J1).

The following table gives the amount of compounds A and B which have been used for the preparation of the composition A2 - J2.

| Composition | Compound A (g) | Compound B (g) |
|---|---|---|
| A2 - G2 | 4 | 96 |
| H2 | 2 | 48 |
| I2 | 3 | 72 |
| J2 | 3 | 72 |

### Preparation of Galenic formulations

### Formulation K1

500 g Nitazoxanide in powder has been mixed with 10 g polyvinylpyrrolidone, 20 g carboxymethyl starch, 25 g corn starch, 5 g magnesium stearate and 50 g water, and the so obtained mixture was then formed into granules of 560 mg (i.e. granules containing 500 mg of active agent). The granules which had a diameter of about 1 cm were then dried at about 50° C.

The so obtained microgranules were then provided with a coating obtained by spraying a hot sugar solution. The sugar coating formed a membrane.

### Formulation K2

A powder mixture of 480g Nitazoxanide (PH5776) and 20g of compound A has been mixed with 10 g polyvinylpyrrolidone, 20 g carboxymethyl starch, 25 g corn starch, 5 g magnesium stearate and 50 g water, and the so obtained mixture was then formed into granules of 560 mg (i.e. granules containing 500 mg of active agent). The granules which had a diameter of about 1 cm were then dried at about 50° C.

The so obtained microgranules were then provided with a coating obtained by spraying a hot sugar solution. The sugar coating formed a membrane.

It is obvious that the microgranules may also contain one or several excipients or other active agents, such as microcristalline cellulose (Avicel ® FMC), methylcellulose, ethylcellulose, carboxymethylcellulose, hydroxyethylcellulose, hydroxypropylcellulose, starch, etc.

In the same way, the membrane may also comprise :
* a pharmacological acceptable excipient such as a plastifiant, a pigment, a filler, a wetting agent, a lubricant,.... or a mixture thereof, and
* a film forming composition which comprises a substance insoluble in the gastric acid but entero soluble (polymeric substance or not, for example cellulose acetophthalate, polyvinyl acetophthalate, hydroxypropylmethylcellulose,...).

### TESTS

### TESTS 1

During phase I, pharmacokinetics study was carried out on 6 volunteers who received a single 500 mg oral dose of the composition 0. Approximately 3 mcg/ml of 2- (hydroxy)-N-(5-nitro-2-thiazolyl) benzamide could be assayed in the blood by High Pressure Liquid Chromatography. This product was also excreted unchanged in urine during the first 24 hours following treatment. There is no trace of-2-(acetolyloxy)-N-(5-nitro 2-thiazolyl) benzamide in blood and urine.

During Phase II clinical studies conducted in a total of 80 patients, post-treatment repeated fecal examinations and/or vaginal smears revealed that 500 mg of the composition A1 given twice a day for 3 to 7 consecutive days was highly effective (60-95 %) against Trichomonas vaginalis, Entamoeba histolytica, Gardia lamblia, Enterobius vermicularis, Ascaris lumbricoides, Necator americanus, Ancylostoma duodenale, Trichuris trichiura, Strongyloides stercoralis, Taenia saginata, Taenia solium, Diphylobottrium latum and Hymenolepis nana. Tolerance was good and only a few epigastric pains, nausea, vomiting and diarrhea were observed in about 10 % of patients depending on the duration of treatment. Blood chemistry and hematology carried out before and after treatment remained unafected by the composition.

In vitro studies against Trichomonas vaginalis has shown that while 2-(acetolyloxy)-N-(5-nitro-2-thiazolyl) benzamide had a Minimum Inhibitory Concentration of 0.5 to 1.25 mcg/ml, 2-(hydroxy)-N-(5-nitro-2-thiazolyl) benzamide in the same experimental conditions showed 1 to 1.25 mcg/ml. This is demonstrating that 2-(hydroxy)-N-(5-nitro-2-thiazolyl) benzamide has an antiparasitic activity equivalent to that of 2- (acetolyloxy) -N- (5-nitro-2-thiazolyl) benzamide. However, these studies showed that 2(hydroxy)-N-(5-nitro 2-thiazolyl) had a substantially immediate action, which was not the case for 2-(acetolyloxy)-N-(5-nitro 2-thiazolyl) benzamide).

Finally in vitro studies have shown that the composition was effective against gram positive and gram negative aerobic bacteria such as Staphylococcus aureus, Escherichia coli, Shigella sonei, Helicobacter pylori ; anaerobic bacteria such as Bacteroides fragilis, Fusobacterium ulcerans, Veillonella alcadescens, Gardnerella vaginalis, dermatophytes of yeasts fungi such as Trichophyton mentographytes, Microsporum audovini, Epidermophyton Flocosum and Candida albicans.

By using a compound of formula I 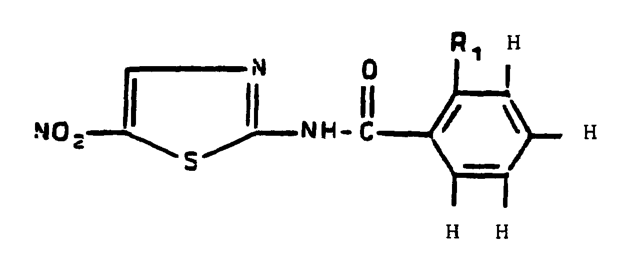 in which the symbol R₁ represents OH,
even in very low amount, it was possible to increase the efficiency of compound PH 5776 (compound B).

### TESTS 2 AND 3

In order to show the effectiveness of the composition according to the invention, two groups of 5 mice (4-8 weeks old; 18-20 g weight each) were tested, both groups being infected with Cryptosporidium parvum.

The infected mice suffered of chronic diarrhea.

Before the treatment, it was easy to determine the mice suffering of chronic diarrhea by fecal analysis.

The treatment takes place by using the composition D1 of nitazoxanide disclosed herebefore. The mice suffering of chronic diarrhea received by oral gavage during one week, each day about 0.01 g of nitazoxanide (i.e. about 0.6 g/kg mice).

After one week of treatment, it was no more possible to determine by fecal analysis which group of mice suffered firstly of diarrhea.

Infected mice were also treated by using the composition D2. Said mice received by oral gavage during one week, each day about 0.0096 g nitazoxanide and about 0.0004 g of compound A of formula III.

Before the end of one week of treatment, it was no more possible to determine by fecal analysis which group of mice suffered firstly of diarrhea.

### TESTS 4 AND 5

Aqueous compositions (100 g) containing 10 g of an active agent have been prepared.

For the composition containing nitazoxanide or a mixture of nitazoxanide (9.6 g) and compound A as active agent, the composition contained furthermore 0.5 g saccharose distearate.

The compositions have been used for treating various parasites, helminths, bacteria and fungus.

The activity of the compositions is given in the following table :

| | ACTIVITY OF | | | | |
|---|---|---|---|---|---|
| | P1 | P2 | P3 | P4 | P5 |
| Protozoa | | | | | |
| Trichomonas vaginalis | + | + | No | No | + |
| Trichomonas intestinalis | + | + | No | No | + |
| Encamoeba histolytica | + | + | No | No | + |
| Encamoeba dispar | + | + | No | No | + |
| Entamoeba coli | + | + | No | No | + |
| Endolimax nana | + | + | No | No | + |
| Balancidium coli | + | + | No | No | + |
| Diencamoeba fragilis | + | + | No | No | + |
| Giarda lamblia | + | + | No | No | + |
| Isospora belli | + | + | +/- | No | + |
| Cryptosporidium parvum | + | + | No | No | No |
| Bladtocystis hominis | + | + | No | No | No |
| Enterocytozoon | | | No | No | No |
| bieneusi | + | + | No | No | No |
| Septata intestinalis | + | + | No | No | No |
| | | | | | |

| Helminths | | | | | |
|---|---|---|---|---|---|
| Enterobius | | | | | |
| vermicularis | + | + | + | + | No |
| Ascaris lumbricoides | + | + | + | + | No |
| Necator americanus | + | + | + | + | No |
| Anycylosloma duodenale | + | + | + | + | No |
| Trichuris trichiura | + | + | + | + | No |
| Strongyloïdes stercoralis | + | + | No | No | No |
| Taenia saginata | + | + | No | No | No |
| Taenia solium | + | + | No | No | No |
| Hymenolepis nana | + | + | No | No | No |
| P1 = Nitazoxanide+ saccharose distearate | | | | | |
| P2 = Nitazoxanide + Compound A + saccharose distearate | | | | | |
| P3 = Albendazole | | | | | |
| P4 = Mebendazole | | | | | |
| P5 = Metronidazole | | | | | |

| ACTIVITY OF | | | | | |
|---|---|---|---|---|---|
| | P1 | P2 | P3 | P4 | P5 |
| Bacteria aerobic | | | | | |
| Staphylococcus aureus | + | + | No | No | No |
| Escherichia coli | + | + | No | No | No |
| Protens vulgaris | + | + | No | No | No |
| | | | | | |

| Bacteria anaerobic | | | | | |
|---|---|---|---|---|---|
| | | | | | |
| Clostridum species | + | + | No | No | + |
| Bacceroides species | + | + | No | No | + |
| Peptococcus species | + | + | No | No | + |
| Peptostreptococcus SPP | | | | | |
| Fusobacterium SPP | + | + | No | No | + |
| | + | + | No | No | |
| | | | | | + |

| Fungus | | | | | |
|---|---|---|---|---|---|
| Candida Albicans | + | + | No | No | No |
| Trychophyton | | | | | |
| Mentagrophytes | + | + | + | No | No |
| Microsporum audovini | + | + | + | No | No |
| Epidermophyton flocosum | + | + | + | No | No |
| P1 = Nitazoxanide + saccharose distearate | | | | | |
| P2 = Nitazoxanide + Compound A + saccharose distearate | | | | | |
| P3 = Albendazole | | | | | |
| P4 = Mebendazole | | | | | |
| P5 = Metronidazole | | | | | |

### TEST 5

General procedures for determining Antiviral Efficiency and Toxicity are given hereafter.

### Laboratory Procedures for Determining Antiviral Efficacy and Toxicity

### A. Preparation of Human Foreskin Fibroblast Cells

Newborn human foreskins were obtained as soon as possible after circumcisions were performed and placed in minimal essential medium (MEM) containing vancomycin, fungizone, penicillin, and gentamycin, at the usual concentrations, for four hours. The medium was then removed, the foreskins minced into small pieces and washed repeatedly until red cells were no longer present. The tissue was then trypsinized using trypsin at 0.25% with continuous stirring for 15 minutes at 37°C in a CO₂ incubator. At the end of each 15 minutes period the tissue was allowed to settle to the bottom of the flask. The supernatant containing cells was poured through sterile cheesecloth into a flask containing MEM and 10 % fetal bovine serum. The flask containing the medium was kept on ice throughout the trypsinizing procedure. After each addition of cells, the cheese cloth was washed with a small amount of MEM containing serum. Fresh trypsin was added each time to the foreskin pieces and the procedure repeated until no more cells became available. The cell containing medium was then centrifuged at 1000 RPM at 4°C for ten minutes. The supernatant liquid was discarded and the cells resuspended in a small amount of MEM with 10 % FBS (Fetal Bovine Serum). The cells were then placed in an appropriate number of 25 cm² tissue culture flasks. As cells became confluent and needed trypsinization, they were gradually expanded into larger flasks. The cells were kept on vancomycin and fungizone to passage four.

### B. Cytopathic Effect Inhibition Assay - HSV, HCMV, VZV

Low passage human foreskin fibroblast cells were seeded into 96 well tissue culture plates 24 hours prior to use at a cell concentration of 2.5 x 10⁴ cells per ml in 0.1 ml of minimal essential medium (MEM) supplemented with 10 % fetal bovine serum (FBS). The cells were then incubated for 24h at 37°C in a CO₂ incubator. After incubation, the medium was removed and 100 µl of MEM containing 2 % FBS was added to all but the first row. In the first row, 125 µl of experimental drug was added in triplicate wells. Medium alone was added to both cell and virus control wells. The drug in the first row of wells was then diluted serially 1:5 throughout the remaining wells by transferring 25 µl using the Cetus Liquid Handling Machine. After dilution of drug, 100 µl of the appropriate virus concentration was added to each well, excluding cell control wells which received 100 µl of MEM. For HSV-1 and HSV-2 assays, the virus concentration utilized can be 1000 PFU's per well. For CMZ and VZV assays, the virus concentration added can be 2500 PFU's per well. The plates were then incubated at 37°C in a CO₂ incubator for three days for HSV-1 and HSV-2, or 10 days for VZV, or 14 days for CMV. After the incubation period, media was aspirated and the cells stained with a 0.1% crystal violet solution for 30 minutes. The stain was then removed and the plates rinsed using tap water until all excess stain was removed. The plates were allowed to dry for 24h and then read on a Skatron Plate Reader at 620 nm.

### C. Plaque Reduction Assay for HSV-1 and HSV-2 using Semi-Solid Overlay

Two days prior to use, HFF (Human Foreskin Fibro blast) cells are plated into six well plates and incubated at 37°C with 5% CO₂ and 90% humidity. On the date of assay, the drug is made up at twice the desired concentration in 2x MEM and then serially diluted 1 : 5 in 2x MEM and then serially diluted 1:5 in 2x MEM using six concentrations of drug. The initial starting concentration is usually 200 µg/ml down to 0.06 µg/ml. The virus to be used is diluted in MEM containing 10 % FBS to a desired concentration which will give 20-30 plaques per well. The media is then aspirated from the wells and 0.2 ml of virus is added to each well in duplicate with 0.2 ml of media being added to drug toxicity wells. The plates are then incubated for one hour with shaking every fifteen minutes. After the incubation period, an equal amount of 1 % agarose was added to a equal volume of each drug dilution. This will give final drug concentrations beginning with 100 µg/ml and ending with 0.03 µg/ml and a final agarose overlay concentration of 0.5 %. The drug agarose mixture is applied to each well in 2 ml volume and the plates then incubated for three days, after which the cells were stained with a 1.5 % solution of neutral red. At the end of 4-6hr incubation period, the stain is aspirated, and plaques counted using a stereomicroscope at 10x magnification.
EC₅₀ (50 % effective concentration) is the concentration required to inhibit viral cytopathogenicity by 50 %.
IC₅₀ (50 % inhibitory concentration) is the concentration required to inhibit cell proliferation by 50 %.
Selective Index (S.I.) = IC₅₀/EC₅₀.

### D. VZV Plaque Reduction Assay - Semi-Solid Overlay

The procedure is essentially the same as for the HSV plaque assay described above with two exceptions :
1. After addition of the drug, the plates are incubated for ten days.
2. On days three and six an additional 1 ml overlay with equal amounts of 2x MEM and 1 % agarose are added.

### E. CMV Plaque Assay - Semi-Solid Overlay

The procedure again is nearly the same as for HSV with a few minor changes. The agarose used for both the initial overlay and the two subsequent overlays is 0.8 % rather than 1 %. The assay is incubated for 14 days with the additional 1 ml overlays being applied on days four and eight.

### F. Plaque Reduction Assays Using Liquid Medium Overlay

The procedure for the liquid overlay plaque assay is similar to that using the agarose overlay. The procedure for adding the virus is the same as for the regular plaque assay. The drugs area concentration to be used in MEM with 2 % FBS. The drugs are not made up at 2x concentration as in the previous assays but are made up at the desired concentration. For HSV-1 and HSV-2 assays, an antibody preparation obtained from Baxter Health Care Corporation is diluted 1:500 and added to the media that the drug is diluted in. For CMV and VZV, no antibody in the overlay is utilized. For the CMV assay, additional medium without new drug is added on day five and allowed to incubate for a total of 10 days. For VZV, additional media is added on day five and incubated for a total of 10 days. At the end of the incubation period for all of the assays, 2 ml of a 1:10 dilution of stock neutral is added to each well and incubated for six hours. The liquid is then aspirated off and plaques enumerated using a stereomicroscope.

### G. Screening and Confirmation Assays for EBV

### 1. Virus

There are two prototypes of infectious EBV. One is exemplified by the virus derived from supernatant fluids of the P3HR-1 cell line. This cell line produces nontransforming virus that causes the production of early antigen (EA) after primary infection or suiperinfection of B cell lines. The other prototype is exemplified by the B-95-8 virus. This virus immortalized cord blood lymphocytes and induced tumors in marmosets. It does not, however, induce an abortive productive infection even in cell lines harboring EBV genome copies. The virus used in our assays is P3HR-1.

### 2. Cell Lines

Ramos is an exceptional B cell line derived from Burkitt's lymphoma tumor but containing no detectable EBV genome copies and is EBNA negative. Ramos/AW was obtained by in vitro infection of Ramos with the P3HR-1 virus and contains one resident EBV genome copy/cell. Raji is a Burkitt's lymphoma cell line containing 60 EBV genomes/cell, and will be the primary cell used for screening antiviral activity against EBV EA expression. Daudi is a low level producer that contains 152 EBV genome copies/cell. It spontaneously expresses EBV EA in 0.25%-0.5 % of the cells. It will be used in follow-up studies to confirm activity. These cell lines respond to superinfection by EBV by expressing EA(D), EA(R), and VCA. All cell lines are maintained in RPMI-1640 medium supplemented by 10% FCS, L-glutamine and 100 µg/ml gentamicin. The cultures are fed twice weekly and the cell concentration adjusted to 3 x 10⁵/ml. The cells are kept at 37°C in an humidified atmosphere with 5% CO₂.

### 3. Immunofluorescence Assays with Monoclonal Antibodies

Cells are infected with the P3HR-1 strain of EBV and the drugs to be tested are added after adsorption (45 minutes at 37°C) and washing of the cell cultures. The cultures are incubated for two days in complete medium to allow viral gene expression. Following the 48 hours incubation period, the number of cells of each sample are counted and smears made. Monoclonal antibodies to the different EA components and VCA are then added to the cells incubated and washed. This is followed by a fluorescein conjugated rabbit anti-mousse Ig antibody ; and, the number of fluorescence positive cells in the smears are counted. The total number of cells in the cultures positive for EA or VCA are then calculated and compared.

### H. Cell Proliferation Assay - Toxicity

Twenty four hours prior to assay, HFF cells are seeded in 6-well plates at a concentration of 2.5 x 10⁴ cells per well in MEM containing 10 % FBS. On the day of the assay, drugs are diluted serially in MEM containing 10 % FBS at increments of 1:5 covering a range from 100 µg/ml to 0.03 µg/ml. For drugs that have to be solubilized in DMSO, control well receive MEM containing 10 % DMSO. The media from the wells is then aspirated and 2 ml of each drug concentration is then added to each well. The cells are then incubated in a CO₂ incubator at 37°C for 72 hours. At the end of this time, the media-drug solution is removed and the cells washed. One ml of 0.25% trypsin is added to each well and incubated until the cells start to come off of the plate. The cell-media mixture is then pipetted up and down vigorously to break up the cell suspension and 0.2 ml of the mixture is added to 9.8 ml of Isoton III and counted using a Coulter Counter. Each sample is counted three times with three replicate wells per sample.

### I. MTT Assay for Cell Cytotoxicity

Twenty-four hours prior to essay, HFF cells are plated into 96 well plates at a concentration of 2.5 x 10⁴, cells per well. After 24 hours, the media is aspirated and 125 microliters of drug is added to the first row of wells and then diluted serially 1:5 using the automated Cetus Liquid Handling System in a manner similar to that used in the CPE assay. The plates are then incubated in a CO₂ incubator at 37°C for seven days. At this time, each well receives 50 microliters of 1 µg/ml solution of MTT in Dulbecco's Phosphate Buffered Saline. The plates are then incubated for an additional four hours. At this time, the media is removed and replaced with 100 µl of 0.04N hydrochloric acid in isopropanol. After shaking briefly, the plates are then read on a plate reader at 550 nm.

### J. Neutral Red Uptake Assay - Toxicity

The procedure for plating cells and adding drug is the same as for MTT Assay.

After drug addition, the plates are incubated for seven days in a CO₂ incubator at 37°C. At this time the media/drug is aspirated and 200 µl/well of 0.01 % neutral red in DPBS is added. This is incubated in the CO₂ incubator for one hour. The dye is aspirated and the cells are washed using a Nunc Plate Washer. After removing the DPBS wash, 200 µg/well of 50 % ETOH (ethanol)/1% glacial acetic acid (in H₂O) is added. The plates are rotated for 15 minutes and the optical densities are read at 550 nm on a plate reader.

### Test5 a : Antiviral activity against HBV replication in cultures (Hepatites B)

The protocol for essaying anti-HBV compounds in cultures of 2.2.15 cells can be briefly summarized as follows (Korba and Milman, 1991, Antiviral Res. 217:217) :
* Chronically HBV-producing human liver cells (Acs, et al, 1987, PNAS 84:4641) are seeded into 24 well tissue culture plates and grown to confluence.
* Test compounds are then added daily for a continuous 9 day period. Culture medium (changed daily during the treatment period) is collected and stored for analysis of extracellular (virion) HBV DNA after 0,3,6 and 9 days of treatment.
* Treated cells are lysed 24 hours following day 9 of treatment for the analysis of intracellular HBV genomic forms.
* HBV DNA is then analyzed in a quantitative and qualitative manner for overall levels of HBV DNA (both extracellular and intracellular DNA) and the relative rate of HBV replication (intracellular DNA).
The protocol for determining toxicity of compounds in cultures of 2.2.15 cells can be briefly summarized as follows (Korba and Gerin, submited for publication) :
* 2.2.15 cells were grown to confluence in 96 well fat-bottomed tissue culture plates and treated with compounds (in 0.2 ml culture medium/well) as described above. Four concentrations of each compound were assayed, each in triplicate cultures, 3 to 10-fold steps.
* Untreated control cultures were maintained on each 96 well plate. On each 96 well plate, wells containing no cells were used to correct for light scattering.
* Toxicity was determined by the inhibition of the uptake of neutral red dye, determined by absorbance at 510 nanometers relative to the absorbance for untreated cells (Finter et al., 1969, J. Med. Chem. 5:419), 24 hours following day 9 of treatment.

The antiviral activity of compound A has been compared with that of zalcitabine.

It was found that when using compound A, the effective concentration (EC 50) was 1.8 ± 0.1 microgram/ml (µg/l) for having a 50% inhibit of viral cytopathogenicity, while the cytotoxic concentration (for having a 50 % inhibit of cell proliferation) was greater than 1000 µg/ml. The selective index SI for compound A was thus equal to >1000/1.8, i.e. a index higher than 500.

For Zalcitabine, the tests showed the following results :

| | |
|---|---|
| EC 50 | 1.8 ± 0.2 µg/ml |
| CC 50 | 261 ± 24 µg/ml |

i.e. a selective index SI of 261/1.8
i.e. about 145.

As it can be seen from this text, Compound A was less toxic than Zalcitabine, whereby a more appropriate treatment with less secondary effects can be obtained against HBV replication.

### TEST 5b : Antiviral activity against VZV

A mixture of Nitazoxanide (96%) and compound A (4%) was also used in order to determine its activity against a Varicella Zoster Virus (a standard laboratory strain).

It was found a EC50 of 4 µg/ml and a CC50 of 34 µg/ml, for the said mixture against said Varicella Zoster Virus, i.e. a S.I. index of about 8.5.

In view of the low toxicity and the efficiency of the mixture, the mixture was particularly suitable for the treatment of Varicella Zoster Virus known as resistant to antiviral agent such as Acyclovir.

Compound A and the composition according to the invention can be administrated orally, for example by means of tablets.

The compositions of the invention, especially those containing PH 5776 and/or a further antiviral agent are compositions having a broad spectrum of action on Herpes viruses such as :
HERPES SIMPLEX VIRUS TYPE 1 (HSV-1, HSV-1 resistant to acyclovir) ;
HERPES SIMPLEX VIRUS TYPE 2 (HSV-S, HSV-2 resistant acyclovir) ;
HUMAN CYTOMEGALOVIRUS (HCMV, HCMV resistant to ganciclovir) ;
VARICELLA ZOSTER VIRUS (VZV, VZV resistant to acyclovir) ;
EPSTEIN BARR BIRUSS (EBV) ;
MURINE CYTOMEGALOVIRUS (MCMV).

The compositions can contain excipients known as such for the purpose of preparing forms suitable for oral administration.

The compositions contain advantageously a wetting agent and possibly a starch derivative.

## Claims

1. Compound of formula 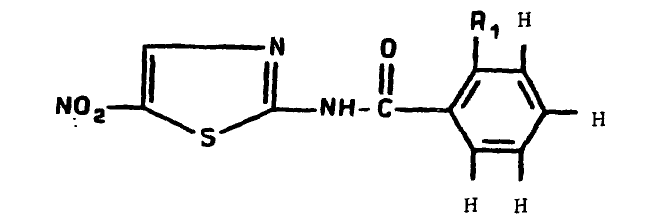 with the symbol R₁ representing OH.

2. Pharmaceutical composition comprising as active agent, the compound of formula according to claim 1 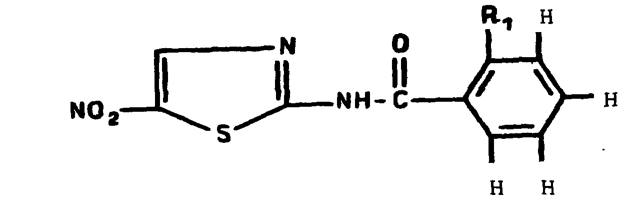 with the symbol R₁ representing OH.

3. Pharmaceutical composition of claim 2, which comprises, as active agent, a mixture of the compound A of formula 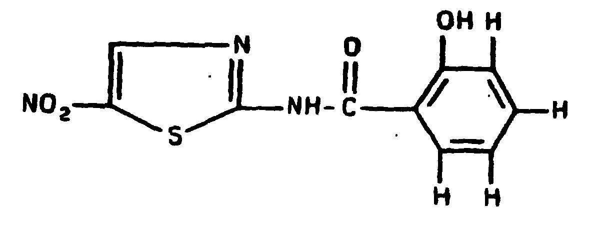 and compound B of formula

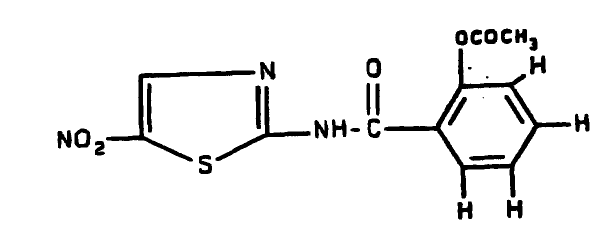

4. Pharmaceutical composition of claim 2 or 3, for oral administration, said composition further containing a wetting agent.

5. Pharmaceutical composition of claim 4, in which at least one wetting agent is selected among the group consisting of the anionic surfactants.

6. Pharmaceutical composition of claim 4, in which the wetting agent is selected among the group consisting of sugar esters, polyoxyethylene, polyoxypropylene, anhydrohexitol derivatives, fatty alkanol amides, fatty amine oxides, sucrose, mannitol, sorbitol, lecithins, polyvinylpyrrolidones, fatty acid esters, glycerides or surcrose, esters of xylose, polyoxyethylene glycerides, esters of fatty acids and polyoxyethylene ether of fatty alcohols and polyoxyethylene fatty acids esters of sorbitan, polyoxyethylene esters of fatty acids of sorbitan, glyceride-polyglycides esters of alcohol polyglycides and mixture thereof.

7. Pharmaceutical composition of anyone of the preceding claims, which contains a starch derivative.

8. Pharmaceutical composition of claim 7, which contains carboxymethyl starch or a salt thereof.

9. The composition of claim 4, which contains up to 20% by weight of surfactant with respect to the weight of active agent(s) and up to 20% by weight of starch derivative with respect to the weight of active agent or agents.

10. Galenic formulation for oral administration, said galenic formulation comprising a core containing a composition containing :
* an effective amount of the compound of formula I according to claim 1 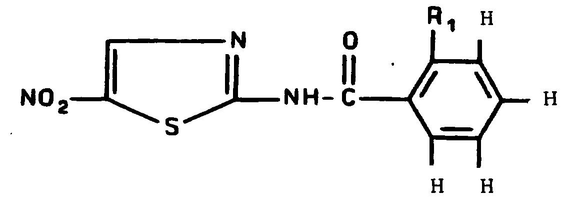 with the symbol R₁ representing OH,
mixed with a compound B of formula 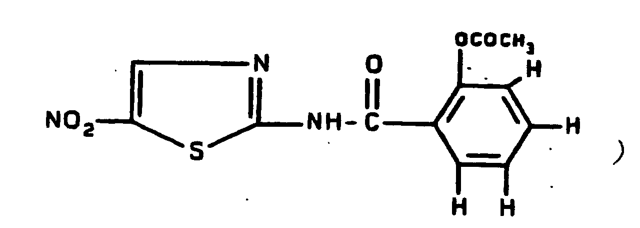 and
* a wetting agent and
* a starch derivative,
the water content of said composition being less than 25% by weight.

11. The formulation of claim 10, containing carboxymethyl starch or a salt thereof.

12. Ointment for the treatment for combatting affections of the lower abdomen, containing :
* an effective amount of active agent of formula I 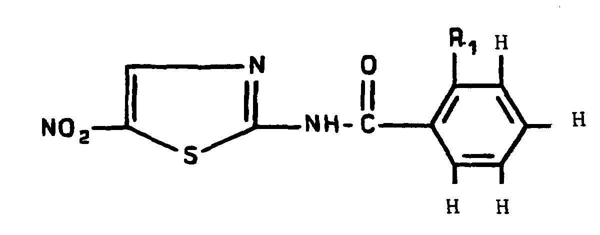 with the symbol R₁ representing OH,
and mixed with a compound B of formula : 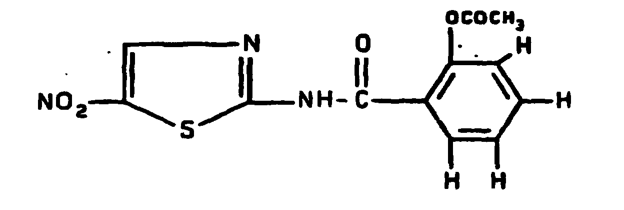 and
* a wetting agent.

13. Ointment of claim 12, which contains a starch derivative

14. Use of the compound of formula I according to claim 1 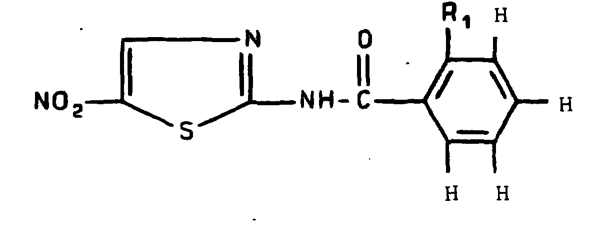 with the symbol R₁ representing OH, in the preparation of an antiparasitic medicament.

15. Use of a mixture of the compound A of formula 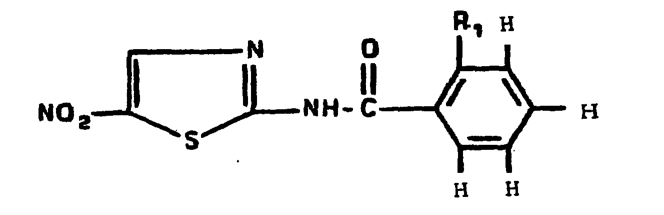 with R₁ = OH, according to claim 1 and a compound B of formula 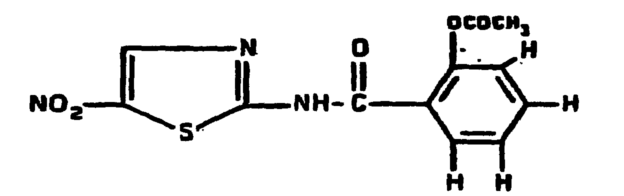 in the preparation of an antiparasitic medicament.

16. Use of the compound of formula I according to claim 1 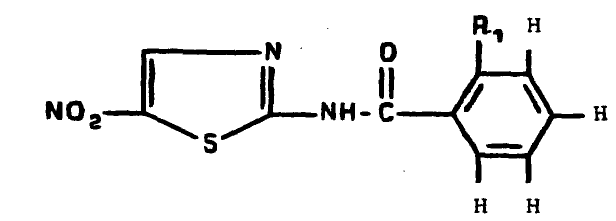 with the symbol R₁ representing OH, or of compound B of formula 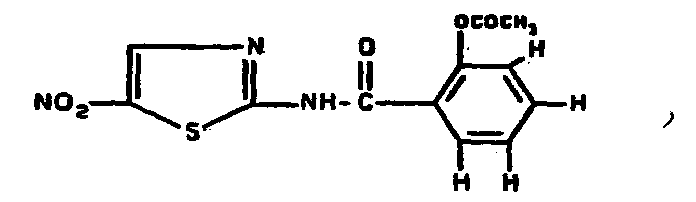 or of a mixture of these compounds, in the preparation of an anti-bacterial medicament.

17. Use according to claim 16, whereby the anti-bacterial medicament is a medicament against bacteria selected from the group comprising Staphylococcus aureus, Escherichia coli, Shigella sonei, Helicobacter pylori, Bacteroides fragilis, Fusobacterium ulcerans, Veillonella alcadescens, Gardnerella vaginalis.

18. Use according to claim 17, whereby the anti-bacterial medicament is active against Helicobacter pylori.

19. Use of the compound of formula I according to claim 1 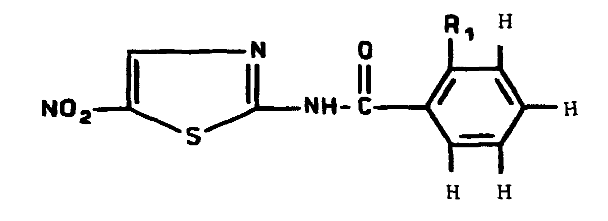 with the symbol R₁ representing OH, in the preparation of an antifungal medicament.

20. Use of a mixture of the compound A of formula 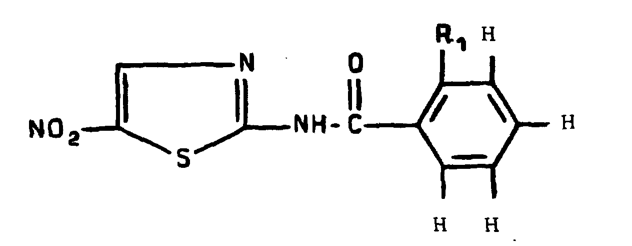 with R₁ = OH, according to claim 1
and of compound B of formula 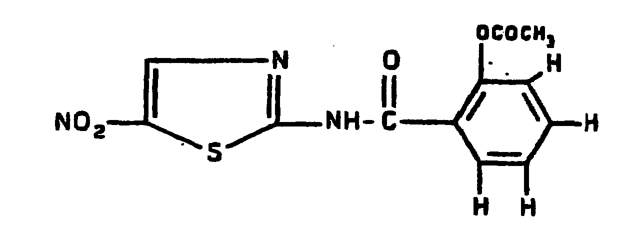 in the preparation of an antifungal medicament.

21. Use of the compound of fomula : 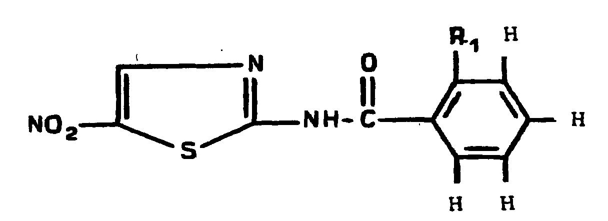 with the symbol R₁ representing OH according to claim 1, or of compound B of formula 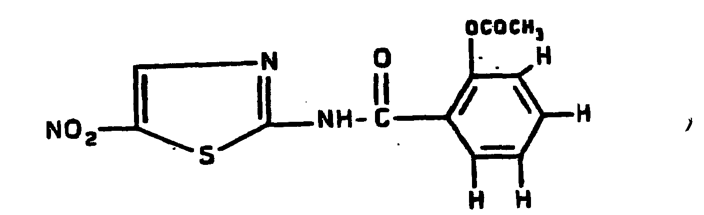 or of a mixture of these compounds in the preparation of an antiviral medicament.

22. Food composition comprising, as preserving agent,
* an effective amount of active agent of formula I, 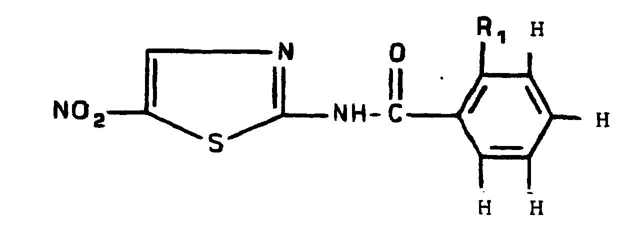 according to claim 1,
with the symbol R₁ representing OH,
* and a compound B of formula 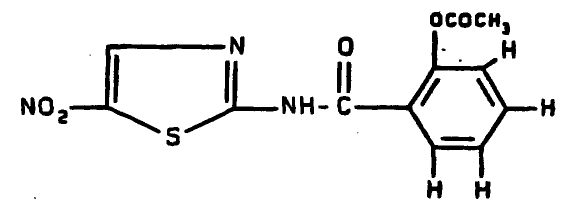
* and a starch derivative.

## Patentansprüche

1. Verbindung der Formel 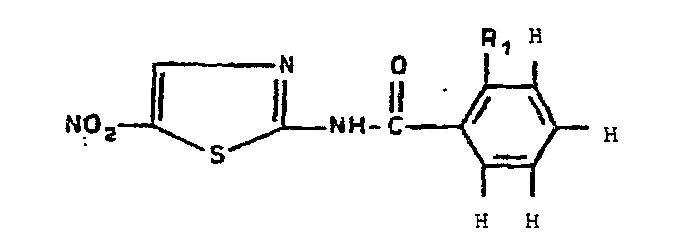 wobei das Symbol R₁ für OH steht.

2. Arzneimittel, umfassend als Wirkstoff die Verbindung der Formel nach Anspruch 1 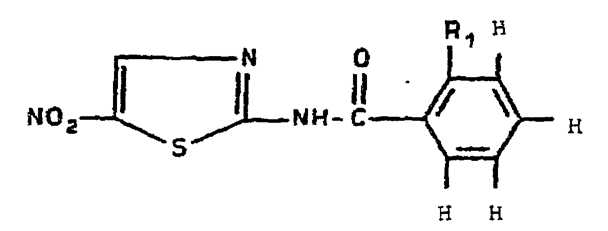 wobei das Symbol R₁ für OH steht.

3. Arzneimittel nach Anspruch 2, umfassend als Wirkstoff ein Gemisch aus der Verbindung A der Formel 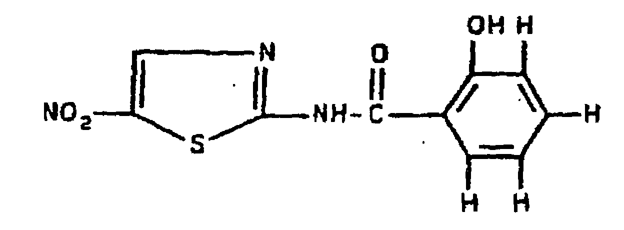 und Verbindung B der Formel

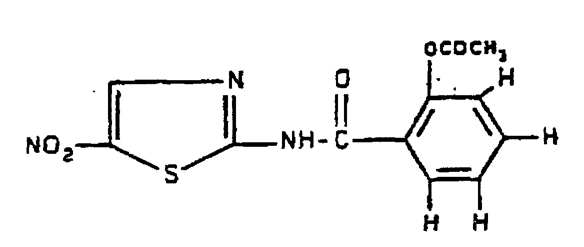

4. Arzneimittel nach Anspruch 2 oder 3 zur oralen Verabreichung, wobei das Mittel zudem ein Benetzungsmittel enthält.

5. Arzneimittel nach Anspruch 4, wobei mindestens ein Benetzungsmittel aus der Gruppe, bestehend aus anionischen grenzflächenaktiven Mitteln, ausgewählt ist.

6. Arzneimittel nach Anspruch 4, wobei das Benetzungsmittel aus der Gruppe ausgewählt ist, bestehend aus Zuckerestern, Polyoxyethylen, Polyoxypropylen, Anhydrohexit-Derivaten, Fettsäurealkanolamiden, Fettsäureaminoxiden, Saccharose, Mannit, Sorbit, Lecithinen, Polyvinylpyrrolidonen, Fettsäureestern, Glyceriden oder Saccharose, Estern von Xylose, Polyoxyethylenglyceriden, Estern von Fettsäuren und Polyoxyethylenethern von Fettalkoholen und Polyoxyethylenfettsäureestern von Sorbitan, Polyoxyethylenestern von Fettsäuren von Sorbitan, Glyceridpolyglycidestern von Alkoholpolyglyciden und Gemischen davon.

7. Arzneimittel nach einem der vorstehenden Ansprüche, das ein Stärkederivat enthält.

8. Arzneimittel nach Anspruch 7, das Carboxymethylstärke oder ein Salz davon enthält.

9. Mittel nach Anspruch 4, das bis zu 20 Gew% grenzflächenaktives Mittel, bezogen auf das Gewicht des (der) Wirkstoff(s/e), und bis zu 20 Gew% Stärkederivat, bezogen auf das Gewicht des Wirkstoffs oder der Wirkstoffe, enthält.

10. Galenische Formulierung zur oralen Verabreichung, wobei die galenische Fonnulierung einen Kern umfasst, der eine Zusammensetzung enthält, die:
eine wirksarne Menge der Verbindung der Formel I nach Anspruch 1 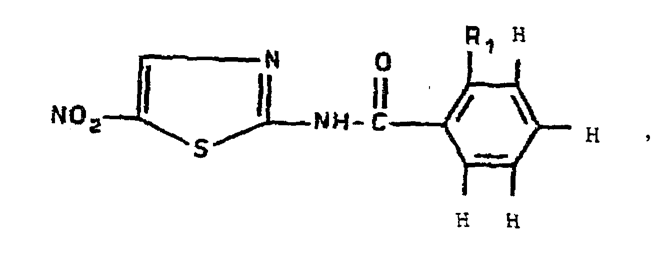
wobei das Symbol R₁ für OH steht,
gemischt mit einer Verbindung B der Formel 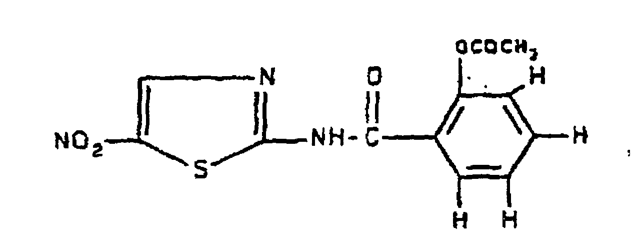 und
- ein Benetzungsmittel und
- ein Stärkederivat enthält,
wobei der Wassergehalt der Zusammensetzung kleiner als 25 Gew% ist.

11. Formulierung nach Anspruch 10, die Carboxymethylstärke oder ein Salz davon enthält.

12. Behandlungssalbe für die Bekämpfung von Erkrankungen des unteren Abdomens, enthaltend:
- eine wirksame Menge Wirkstoff der Formel I 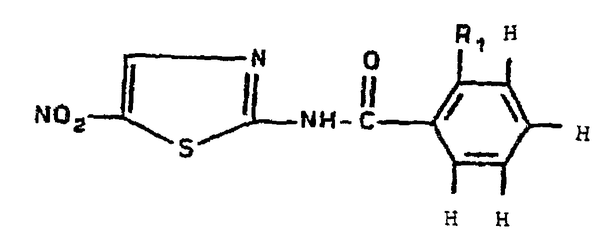 wobei das Symbol R₁ für OH steht,
gemischt mit einer Verbindung B der Formel 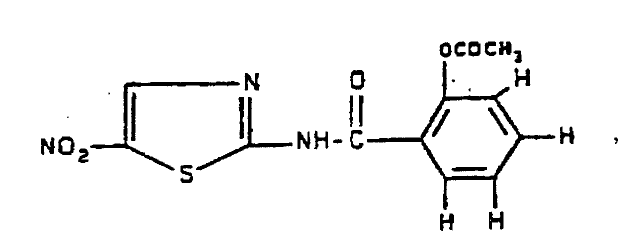 und ein Benetzungsmittel.

13. Salbe nach Anspruch 12, die ein Stärkederivat enthält.

14. Verwendung der Verbindung der Formel I nach Anspruch 1 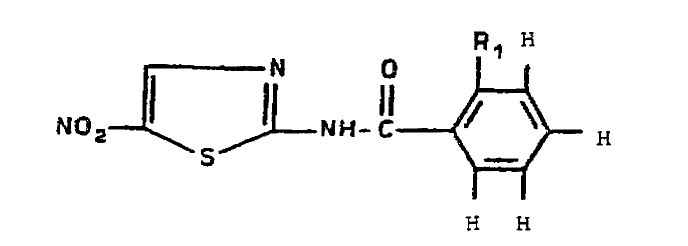 wobei das Symbol R₁ für OH steht, zur Herstellung eines antiparasitischen Medikaments.

15. Verwendung eines Gemischs der Verbindung A der Formel 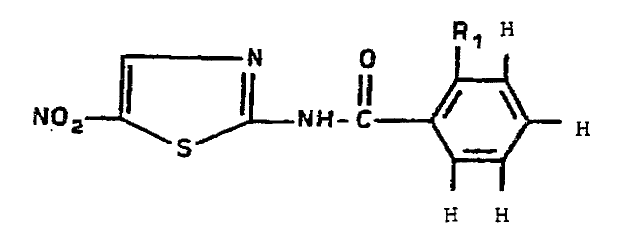 wobei das Symbol R₁ für OH steht, nach Anspruch 1, und einer Verbindung B der Formel 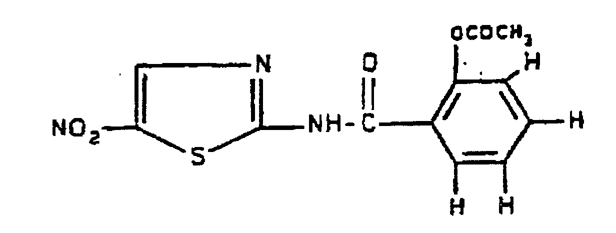 zur Herstellung eines antiparasitischen Medikaments.

16. Verwendung der Verbindung der Formel I nach Anspruch 1 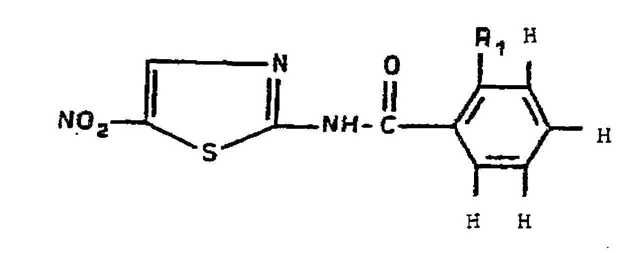 wobei das Symbol R₁ für OH steht, oder von Verbindung B der Formel 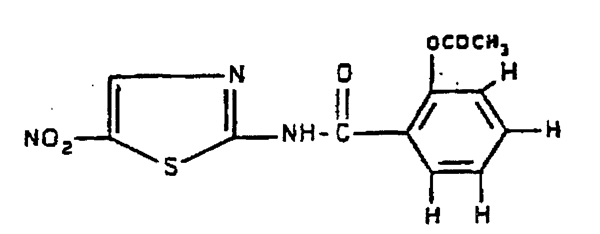 oder eines Gemischs dieser Verbindungen zur Herstellung eines antibakteriellen Medikaments.

17. Verwendung nach Anspruch 16, wobei das antibakterielle Medikament ein Medikament gegen Bakterien ist, die aus der Gruppe, umfassend Staphylococcus aureus, Escherichia coli, Shigella sonei, Helicobacter pylori, Bacteroides fragilis, Fusobacterium ulcerans, Veillonella alcadescens, Gardnerella vaginalis, ausgewählt sind.

18. Verwendung nach Anspruch 17, wobei das antibakterielle Medikament gegen Helicobacter pylori wirksam ist.

19. Verwendung der Verbindung der Formel I nach Anspruch 1 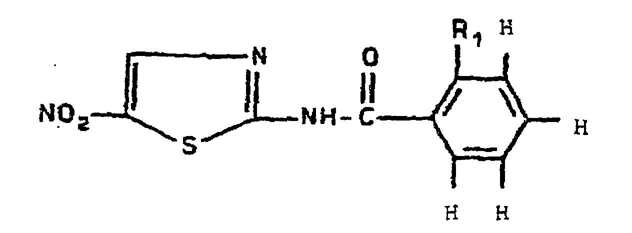 wobei das Symbol R₁ für OH steht, zur Herstellung eines antifungiziden Medikaments.

20. Verwendung eines Gemischs der Verbindung A der Formel 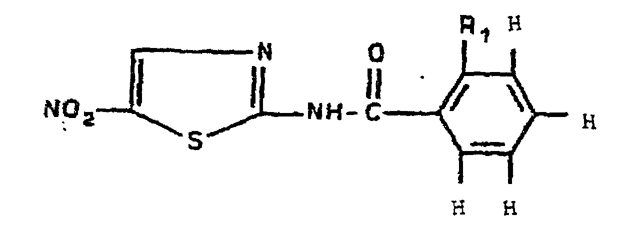 wobei R₁ = OH, nach Anspruch 1, und von Verbindung B der Formel 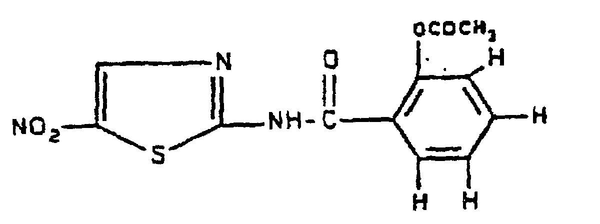 zur Herstellung eines antifungiziden Medikaments.

21. Verwendung der Verbindung der Formel 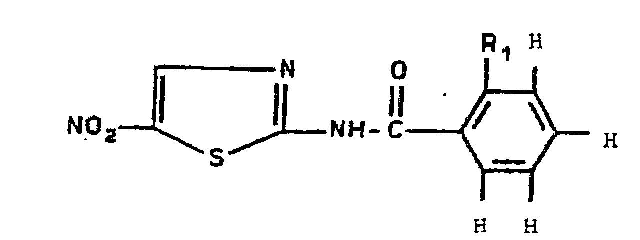 wobei das Symbol R₁ für OH steht, nach Anspruch 1, oder von Verbindung B der Formel 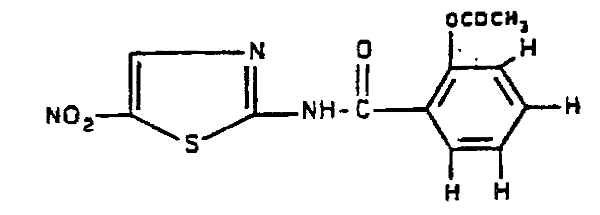 oder eines Gemischs dieser Verbindungen zur Herstellung eines antiviralen Medikaments.

22. Nahrungsmittelzusammensetzung, umfassend als Konservierungsmittel eine wirksame Menge des Wirkstoffs der Formel I 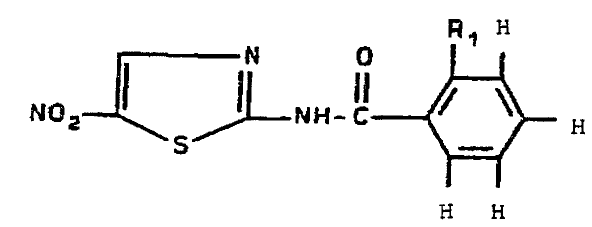 nach Anspruch 1, wobei das Symbol R₁ für OH steht,
- und eine Verbindung B der Formel 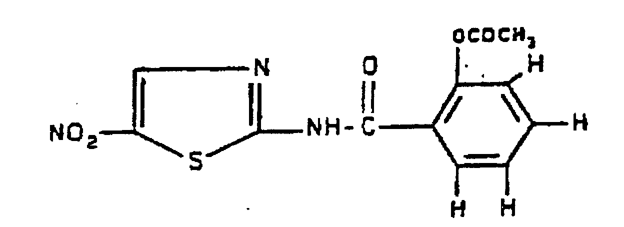
- und ein Stärkederivat.

## Revendications

1. Composé de formule : 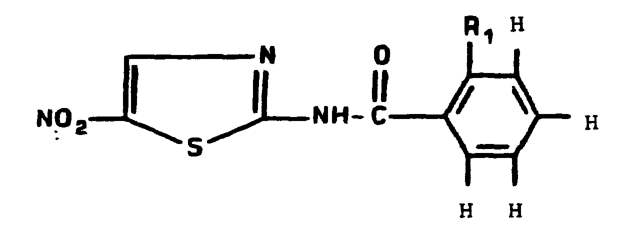 avec le symbole R₁ représentant OH.

2. Composition pharmaceutique comprenant comme agent actif, le composé de formule suivant la revendication 1 : 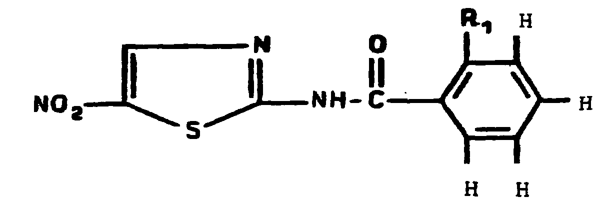 avec le symbole R₁ représentant OH.

3. Composition pharmaceutique suivant la revendication 2, qui comprend, comme agent actif, un mélange du composé A de formule: 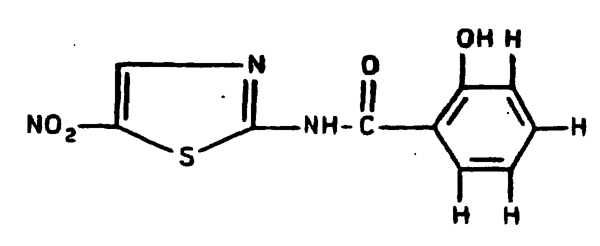 et de composé B de formule :

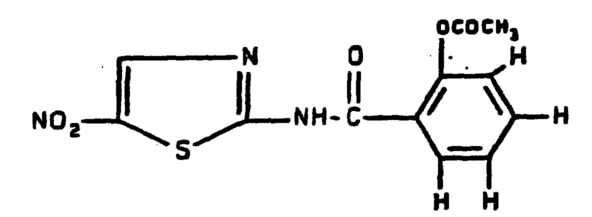

4. Composition pharmaceutique suivant l'une ou l'autre des revendications 2 et 3, pour l'administration orale, ladite composition contenant de plus un agent mouillant.

5. Composition pharmaceutique suivant la revendication 4, dans laquelle au moins un agent mouillant est choisi dans le groupe comprenant les agents tensioactifs anioniques.

6. Composition pharmaceutique suivant la revendication 4, dans laquelle l'agent mouillant est choisi dans le groupe comprenant les esters de sucre, le polyoxyéthylène, le polyoxypropylène, les dérivés d'anhydrohexitol, les alcanolamides gras, les oxydes d'amines grasses, le sucrose, le mannitol, le sorbitol, les lécithines, les polyvinylpyrrolidones, les esters d'acides gras, les glycérides de sucrose, les esters de xylose, les glycérides de polyoxyéthylène, les esters d'acides gras et d'éther polyoxyéthylénique d'alcools gras et les esters d'acides gras polyoxyéthyléniques de sorbitane, les esters polyoxyéthyléniques d'acides gras de sorbitane, les esters de glycéride-polyglycides de polyglycides d'alcools et leurs mélanges.

7. Composition pharmaceutique suivant l'une quelconque des revendications précédentes, qui contient un dérivé d'amidon.

8. Composition pharmaceutique suivant la revendication 7, qui contient de l'amidon carboxyméthylé ou un sel de celui-ci.

9. Composition suivant la revendication 4, qui contient jusqu'à 20 % en poids d'agent tensioactif par rapport au poids d'agent(s) actif(s) et jusqu'à 20 % en poids de dérivé d'amidon par rapport au poids d'agent ou d'agents actifs.

10. Formulation galénique pour administration orale, ladite formulation galénique comprenant un noyau contenant une composition contenant :
* une quantité efficace du composé de formule I suivant la revendication 1 : 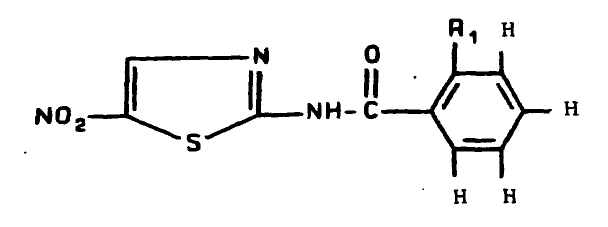 avec le symbole R₁ représentant OH,
mélangé avec un composé B de formule : 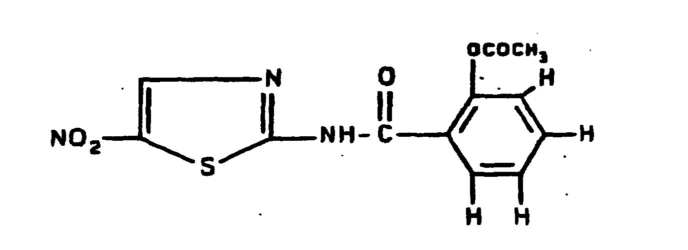 et
* un agent mouillant, et
* un dérivé d'amidon,
la teneur en eau de la composition précitée étant inférieure à 25 % en poids.

11. Formulation suivant la revendication 10, contenant de l'amidon carboxyméthylé ou un sel de celui-ci.

12. Onguent pour le traitement pour combattre les affections de l'abdomen inférieur, contenant :
* une quantité efficace d'agent actif de formule I : 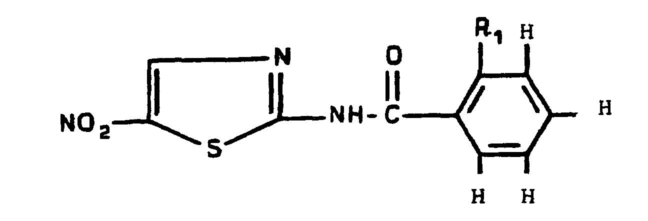 avec le symbole R₁ représentant OH,
et mélangée avec un composé B de formule : 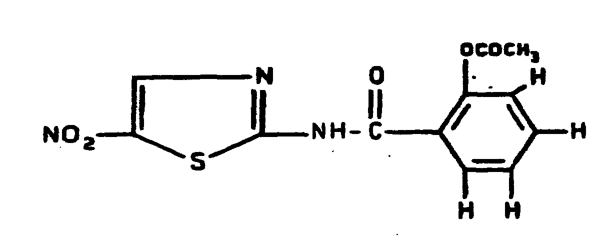 et
* un agent mouillant.

13. Onguent suivant la revendication 12, qui contient un dérivé d'amidon.

14. Utilisation du composé de formule I suivant la revendication 1 : 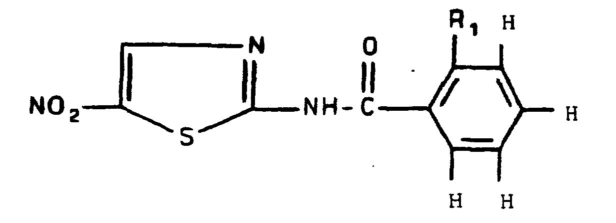 avec le symbole R₁ représentant OH, dans la préparation d'un médicament antiparasitaire.

15. Utilisation d'un mélange du composé A de formule : 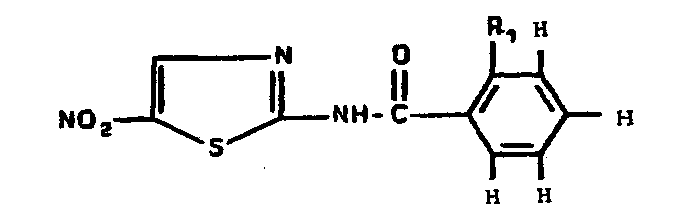 avec R₁ = OH, suivant la revendication 1
et d'un composé B de formule : 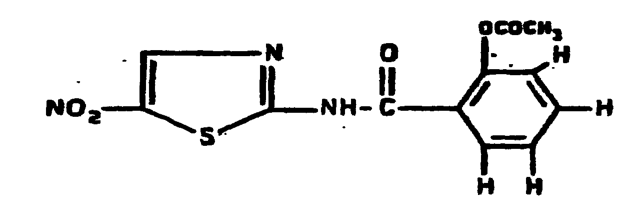 dans la préparation d'un médicament antiparasitaire.

16. Utilisation du composé de formule I suivant la revendication 1 : 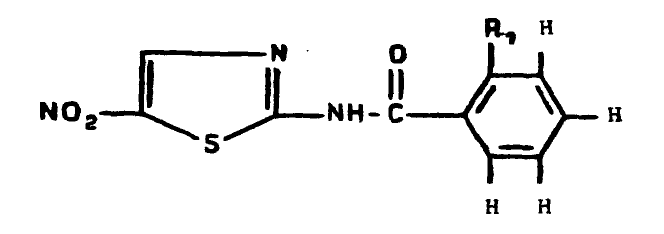 avec le symbole R₁ représentant OH, ou de composé B de formule : 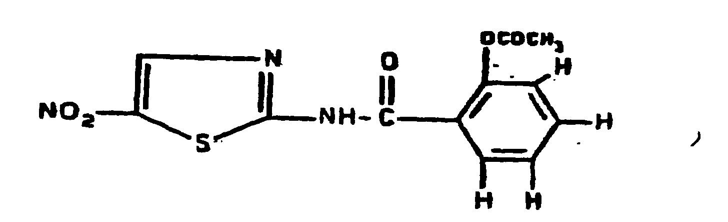 ou d'un mélange de ces composés, dans la préparation d'un médicament antibactérien.

17. Utilisation suivant la revendication 16, le médicament étant un médicament contre les bactéries choisi dans le groupe comprenant Staphylococcus aureus, Escherichia coli, Shigella sonei, Helicobacter pylori, Bacteroides fragilis, Fusobacterium ulcerans, Veillonella alcadescens, Gardnerella vaginalis.

18. Utilisation suivant la revendication 17, le médicament antibactérien étant actif contre Helicobacter pylori.

19. Utilisation du composé de formule I suivant la revendication 1 : 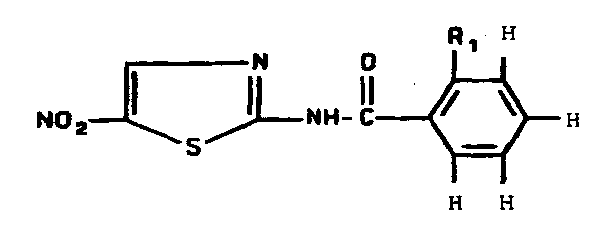 avec le symbole R₁ représentant OH, dans la préparation d'un médicament antifongique.

20. Utilisation d'un mélange du composé A de formule : 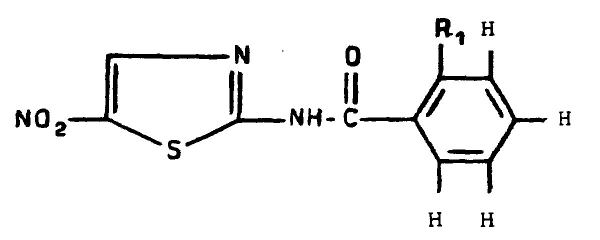 avec R₁ = OH, suivant la revendication 1
et de composé B de formule : 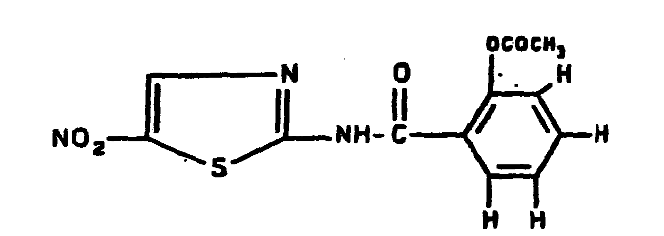 dans la préparation d'un médicament antifongique.

21. Utilisation du composé de formule : 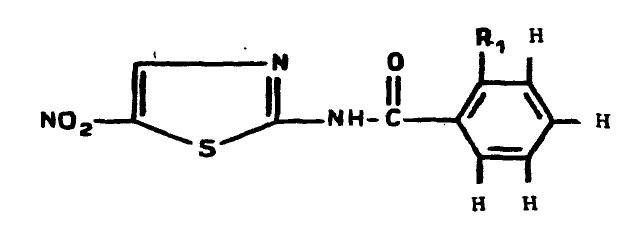 avec le symbole R₁ représentant OH suivant la revendication 1, ou de composé B de formule 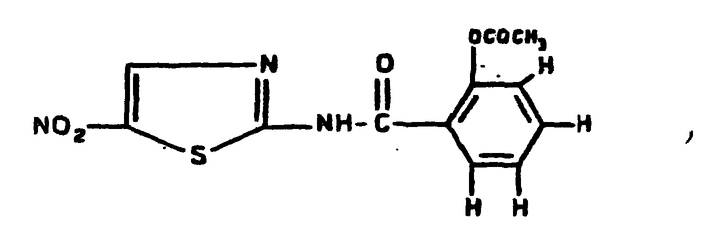 ou d'un mélange de ces composés, dans la préparation d'un médicament antiviral.

22. Composition alimentaire comprenant, comme agent de conservation,
* une quantité efficace d'un agent actif de formule I : 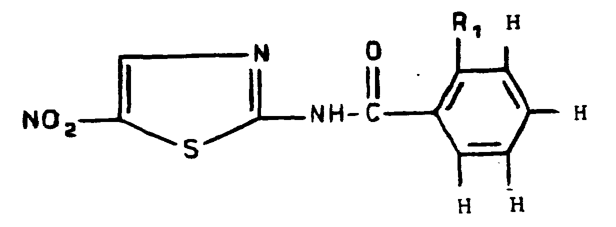 suivant la revendication 1, avec le symbole R₁ représentant OH,
* et un composé B de formule : 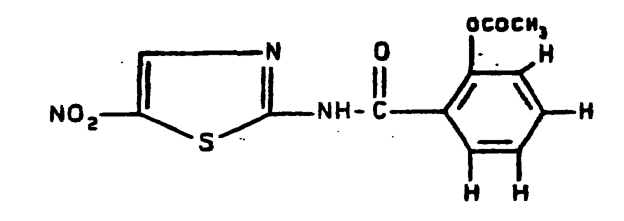
* et un dérivé d'amidon.
